# EUROPEAN PATENT APPLICATION

(11) **EP 1 932 557 A1**
(43) Date of publication of application: **18.06.2008**
(21) Application number: 05788327.4
(22) Date of filing: 03.10.2005
(51) Int. Cl.: A61M 5/178

(54) **MEDICAL DEVICE**

(71) Applicant: OLYMPUS MEDICAL SYSTEMS CORP., Shibuya-ku Tokyo 151-0072 (JP)
(72) Inventor: MATSUMOTO, Kanichi c/o Olympus Medical Systems Corp., Shibuya-ku Tokyo 151-0072 (JP)
(74) Representative: von Hellfeld, Axel
(86) International application number: PCT/JP2005/018303
(87) International publication number: WO 2007/039930

(57) **Abstract**

A medical apparatus includes: a container portion including a space portion filled with a liquid mixed with a bacterium to be injected into a region to be treated, and a gamma-ray shielding portion for shielding gamma rays irradiated in gamma-ray sterilization; a needle tube including a flow passage through which the liquid passes having one end portion in communication with the space portion of the container portion, and the other end portion punctured in the region to be treated; and a pressing operation portion operated in injecting the liquid filled in the space portion of the container portion into the region to be treated.

## Description

### Technical Field

The present invention relates to a medical apparatus for injecting a bacterium to a region to be treated in a biological body.

### Background Art

Conventionally, a diagnosis whether or not a patient is suspected of prostate cancer has been performed by digital rectal examination, prostate-specific antigen, and transrectal ultrasound. When it is judged to be prostate cancer, total prostatectomy for completely removing the prostate has been generally performed. In addition, in a case of prostatic hyperplasia, treatment is performed such that an endoscope is inserted into a urethra to insert a treatment instrument for laser irradiation, or an RF probe via a treatment instrument insertion channel provided to the endoscope and necrotize tissues.

In recent years, as a method of treating prostatic hyperplasia, there is a method of injecting ethanol into a prostate to necrotize the tissues or botulinum having a working to reduce the prostatic hyperplasia. In this case, a needle tube extended from a syringe is inserted through the treatment instrument insertion channel provided to the endoscope to puncture the needle tube into the prostate, thereby injecting ethanol or botulinum. In addition, an overactive bladder, in which contractile action of the bladder frequently occurs, causes urinary incontinence, or frequent urination, i.e., to have a frequent urge to urinate. Also in the method of treating such a case, it is known that the contractile action of the bladder returns to normal by injecting botulinum into a predetermined region of bladder wall.

In a case of performing a procedure to inject botulinum as described above, how to deal with botulinum becomes a problem. That is, during treatment, it is difficult to suck and inject botulinum into a syringe cylinder of a syringe. Therefore, medical workers anticipate that a syringe containing botulinum previously filled in a syringe cylinder is provided stored in a sterile pack. However, if gamma rays used in sterilizing the syringe is irradiated to the botulinum in the syringe cylinder, the botulinum is killed.

For example, Japanese Unexamined Patent Application Publication No. 8-238314 discloses a syringe which does not cause in-hospital infection or medical accident and enables sterilizing processing at the time of production thereof to be easily and surely performed without giving bad influences on an outer cylinder and the like. When the syringe is sterilized by gamma rays, the syringe is stored in a packaging material together with deoxidant in a state where the outer cylinder combining a plunger, a syringe needle, and a protector is remained clamped. Then a storage opening portion of the packaging material is heat-sealed. After the heat seal, the packaging material is covered with a gamma-ray shielding plate to a position of a middle portion of the cylindrical member. Then, in this state, a known gamma-ray sterilization is performed on an outer cylinder portion. After the sterilization is finished, a clamp is removed from the packaging material. Then, the protector and the cylindrical member are connected from outside of the packaging material via a projection portion and a guiding groove. Thus, production of the syringe is completed.

However, in the syringe disclosed in the Japanese Unexamined Patent Application Publication No. 8-238314, the outer cylinder configuring the syringe, a flexible resin container in which injectable solution is sterilized and housed, and the like are stored in the packaging material, so that simplification was difficult. In addition, when performing gamma-ray sterilization, it is necessary to prevent gamma rays from being irradiated to the syringe by using the gamma-ray shielding plate. Accordingly, there is such a problem as sterilization work is troublesome for a worker.

Moreover, when treatment of injecting ethanol or botulinum into the prostate via the needle tube inserted through the treatment instrument insertion channel is performed in order to treat the prostatic hyperplasia, sometimes there is a need for injecting analgesic, activator, or anti-inflammatory agent other than the ethanol or botulinum. In such a case, the needle tube for injecting the analgesic, the activator, or the anti-inflammatory agent and the needle tube for injecting the ethanol or the botulinum are required to be inserted into and extracted from the treatment instrument insertion channel of the endoscope.

The present invention has been achieved in view of the above-mentioned points, and the object of the present invention is to provide a user-friendly medical apparatus that is easy for an operator to inject a bacterium for treatment into a body after taking the medical apparatus out of a sterile pack, and enables a worker who performs sterilization to smoothly perform sterilization processing.

### Disclosure of Invention

### Means for Solving the Problem

A medical apparatus of the present invention comprises: a container portion including a space portion to be filled with a liquid mixed with a bacterium to be injected into a region to be treated, and a gamma-ray shielding portion for shielding gamma rays irradiated in gamma-ray sterilization; a needle tube provided with a flow passage through which the liquid passes, the needle tube including one end portion in communication with the space portion of the container portion, and the other end portion to be punctured in the region to be treated; and a pressing operation portion operated in injecting the liquid filled in the space portion of the container portion into the region to be treated.

### Brief Description of the Drawings

FIG. 1 is a view showing a syringe stored in a sterile bag.
FIG. 2 is a view describing a configuration of the syringe.
FIG 3 is a view describing one configuration of the syringe preventing gamma rays from reaching an inner space filled with medicinal agent.
FIG. 4 is a view describing another configuration of the syringe preventing gamma rays from reaching the inner space filled with the medicinal agent.
FIG. 5 is a view describing a state where an insertion portion of an endoscope is inserted into a urethra.
FIG. 6 is a view describing a state where a needle tube of the syringe is introduced in a prostate via a treatment instrument insertion port.
FIG. 7 is a view describing a state where the needle tube is punctured into the prostate.
FIG. 8 is a view describing a state where the needle tube is punctured into the prostate via a treatment instrument channel provided to an ultrasound endoscope.
FIG. 9 is a view showing a state where the needle tube is displayed on an ultrasonographic image.
FIG. 10 is a view showing a syringe of another configuration stored in a sterile bag.
FIG. 11 is a view describing a syringe in which a container is housed in a container disposing hole of an outer cylinder.
FIG. 12 is a view describing one configuration of the container.
FIG. 13 is an exploded perspective view describing a syringe of another configuration.
FIG. 14 is a view describing the syringe in which the container is housed in a container disposing hole of the outer cylinder.
FIG 15 is a view showing a syringe of another configuration stored in a sterile bag.
FIG. 16 is a view showing the syringe in an assembled state.
FIG 17 is a view describing another configuration of the container.
FIG 18 is a view describing a configuration of an injection apparatus provided with observation means.
FIG. 19 is a cross-sectional view in a longitudinal direction describing a configuration of the insertion portion of the endoscope.
FIG. 20 is a cross-sectional view along XX-XX line of FIG. 19.
FIG. 21 is a perspective view describing an operation portion of the endoscope.
FIG. 22 is a perspective view describing a confiugration of a case body disposing table.
FIG. 23 is an explanatory view including a partly cross-sectional view describing configurations of the case body disposing table and a disposing table moving rod and a relationship therebetween.
FIG. 24 is a view showing the case body disposing table, the disposing table moving rod, and an injection operating rod retained in a puncture waiting state.
FIG. 25 is a view showing the case body disposing table, the disposing table moving rod, and the injection operating rod in a released state of the puncture waiting.
FIG 26 is a view showing a state where the puncture waiting state is released and the needle tube is punctured into the prostate.
FIG. 27 is a view describing a state where medicinal agent is injected into the prostate by pushing-in operation of the injection operating rod.
FIG 28 is a view describing another configuration of the endoscope.
FIG. 29 is a view describing a different configuration of the endoscope.
FIG. 30 is a view describing yet another configuration of the endoscope.
FIG. 31 is a view describing still another configuration of the endoscope and a state where the needle tube of the syringe is inserted in the added treatment instrument channel.
FIG. 32 is a cross-sectional view along XXXII-XXXII line of FIG. 31.
FIG 33 is a view describing the endoscope including an operation portion provided with the case body disposing tables, the disposing table moving rods, and the injection operating rods corresponding to the two treatment instrument channels.
FIG. 34 is a view describing an endoscope including the bending portion in the insertion portion.

### Best Mode for Carrying Out the Invention

Hereinafter, embodiments of the present invention are described with reference to the drawings.

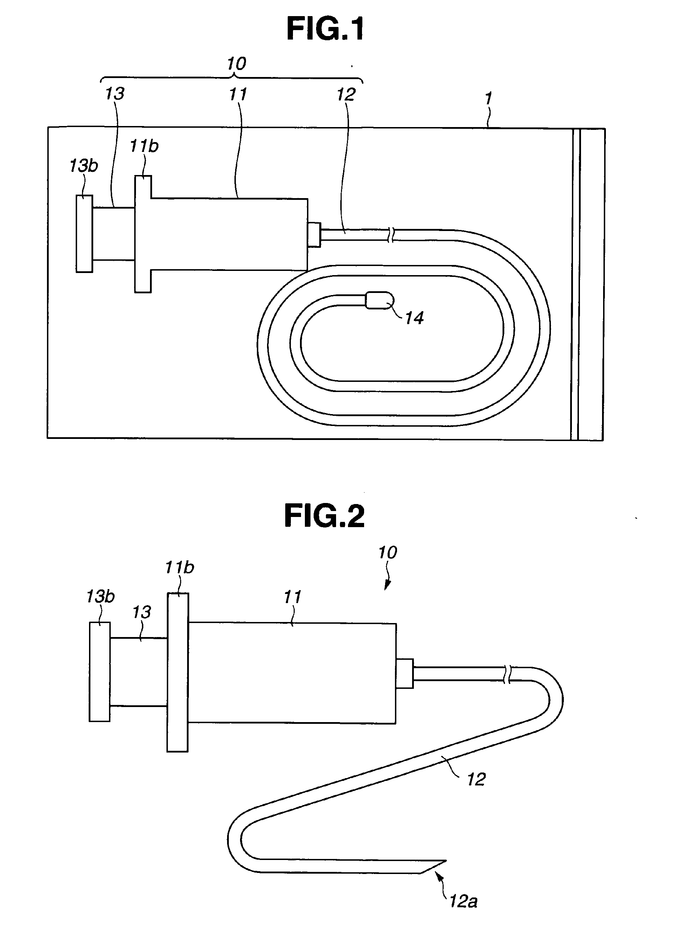

A first embodiment of the present invention is described with reference to FIGS. 1 to 7.

As shown in FIGS. 1 to 3, a syringe 10 as a medical apparatus of the present embodiment is for treating prostatic hyperplasia, for example, and is provided stored in a sterile bag 1 as a packaging material for sterilization. The syringe 10 is mainly configured of an outer cylinder 11, a needle tube 12, and an inner cylinder 13. The needle tube 12 has at a distal end thereof a cap 14 preventing a needlepoint 12a having a sharp-pointed shape from being exposed. The cap 14 also serves as a stopper portion for preventing medicinal agent filled in the outer cylinder 11 from leaking out.

Note that the needle tube 12, as shown in the drawing, is formed of a resin member and the like having a flexibility in a bendable configuration, and is formed of rigid metal member or rigid resin member in a rigid configuration.

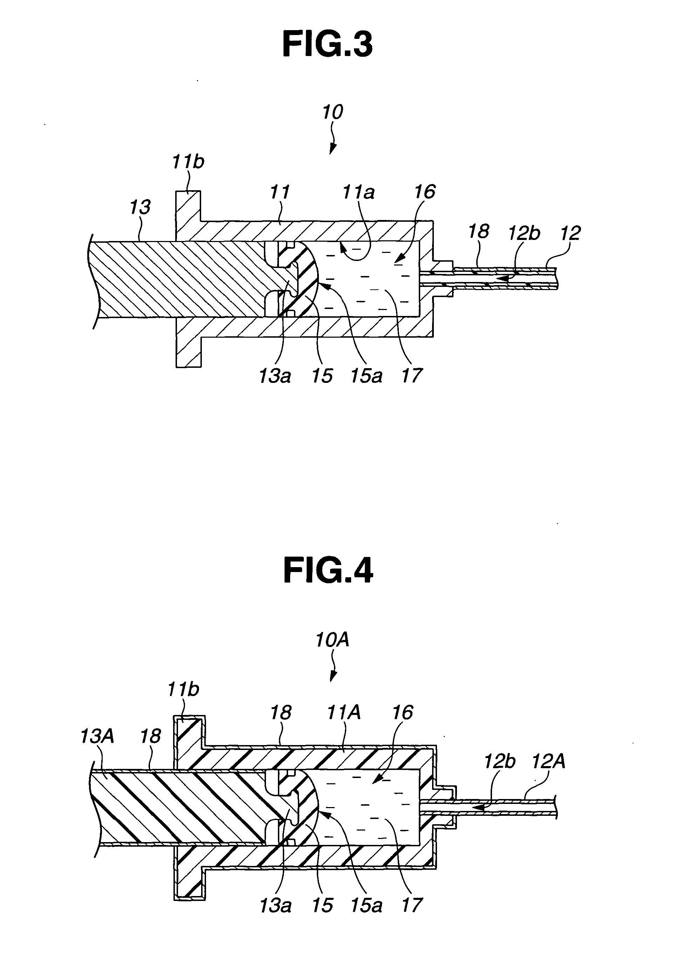

As shown in FIGS. 2, 3, the outer cylinder 11 is a cylindrical container portion and includes a recessed portion 11a. The needle tube 12 includes a flow passage 12b, and a proximal end portion of the needle tube 12 is integrally attached to a distal end portion of the outer cylinder 11 by adhesive bonding and the like. The flow passage 12b of the needle tube 12 is in communication with the recessed portion 11 a of the outer cylinder 11. To the recessed portion 11 a of the outer cylinder 11 is slidably disposed an inner cylinder 13. The inner cylinder 13 is a pressing operation portion and includes at a distal end portion thereof a stepped protrusion portion 13a. The stepped protrusion portion 13a is provided with a rubber valve 15.

Note that reference numerals 11b and 13b represent a projection portion for holding and a projection portion for pushing-in operation, respectively. On the projection portion for holding 11b are placed, for example, fingers other than the thumb of an operator who holds the syringe 10. On the projection portion for pushing-in operation 13b is placed, for example, the thumb of the operator who holds the syringe 10.

In the present embodiment, a medicinal agent 17 is filled in advance in an inner space 16 configured by a distal end-side surface 15a of the valve 15 provided to the stepped protrusion portion 13a of the inner cylinder 13 and the recessed portion 11 a of the outer cylinder 11. The medicinal agent 17 is produced by mixing the botulinum which is a bacterium having a working of treating the prostatic hyperplasia in a predetermined liquid, for example. The syringe 10 filled with a predetermined amount of the medicinal agent 17 is subjected to the gamma-ray sterilization in a state of being housed in the sterile bag 1, as shown in FIG. 1.

When the botulinum mixed in the medicinal agent 17 is irradiated with gamma rays, the botulimum is killed. Therefore, the syringe 10 is provided with a gamma-ray shielding portion for preventing the gamma rays from reaching the medicinal agent 17 and killing the botulinum mixed in the medicinal agent 17 during the gamma-ray sterilization. Specifically, the gamma-ray shielding portion in the syringe 10 is a gamma-ray shielding member for shielding the gamma rays, and is the outer cylinder 11 and the inner cylinder 13 configured of metal members such as stainless-steel member and the like, for example. In addition, when the needle tube 12 is configured of a resin member, for example, the needle tube 12 is provided on outer surface thereof with a coating layer 18 formed by the gamma-ray shielding member as the gamma-ray shielding portion.

In a syringe 10A shown in FIG. 4, an outer cylinder 11A and an inner cylinder 13A are configured of transparent resin members, for example. When the outer cylinder 11A and the inner cylinder 13A are configured of the resin members, the coating layer 18 formed by the gamma-ray shielding member is provided on at least one of the outer surface and the inner surface of the outer cylinder 11 A and on at least one of the outer surface and the inner surface of the inner cylinder 13A.

Note that a needle tube 12A configuring the syringe 10A is formed to be rigid by the metal member for shielding gamma rays. Other configurations are the same as those of the syringe 10. The same members are attached with the same reference numerals and descriptions thereof are omitted.

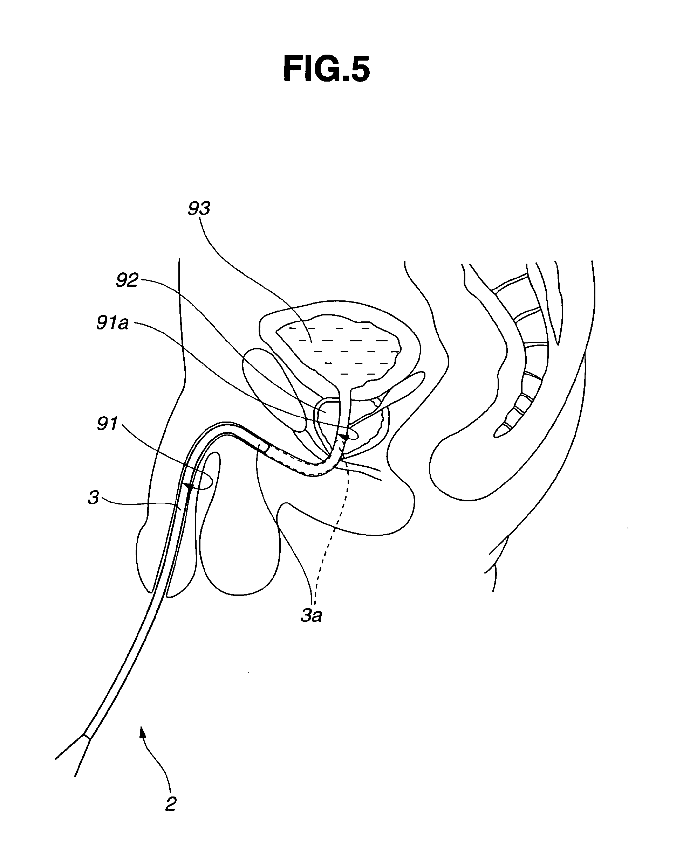

With reference to FIGS. 5 to 7, description is made on transurethral treatment of prostatic hyperplasia using the syringe 10.

When performing transurethral treatment of prostatic hyperplasia, an operator reviews in advance preoperative images such as a CT image, an MRI image, and the like, as needed, to grasp the state of the prostate, and determines a plurality of medicinal agent injecting positions. Meanwhile, stuff members prepare the endoscope 2 and the syringe 10 stored in the sterile bag 1.

At the time of treatment, the operator inserts the insertion portion 3 of the endoscope 2 into a urethra 91, as shown in FIG. 5. Then the operator inserts the distal end portion 3a of the insertion portion 3 toward a prostate 92 which is a region to be treated while observing an endoscope image picked up by an observation optical system provided to the endoscope 2 and displayed on a screen of a monitor not shown. Note that the reference numeral 93 represents a bladder.

As shown by the dashed line of FIG 5, the insertion portion 3 is inserted into the prostate 92. At this time, the operator confirms whether or not the distal end portion 3a has reached the indicator determined in advance from the endoscope image displayed on the screen of the monitor. When confirming the distal end portion 3a has reached a target region, the operator performs bending operation and the like, as needed, to bring the distal end surface of the distal end portion 3a to face a urethral wall 91a.

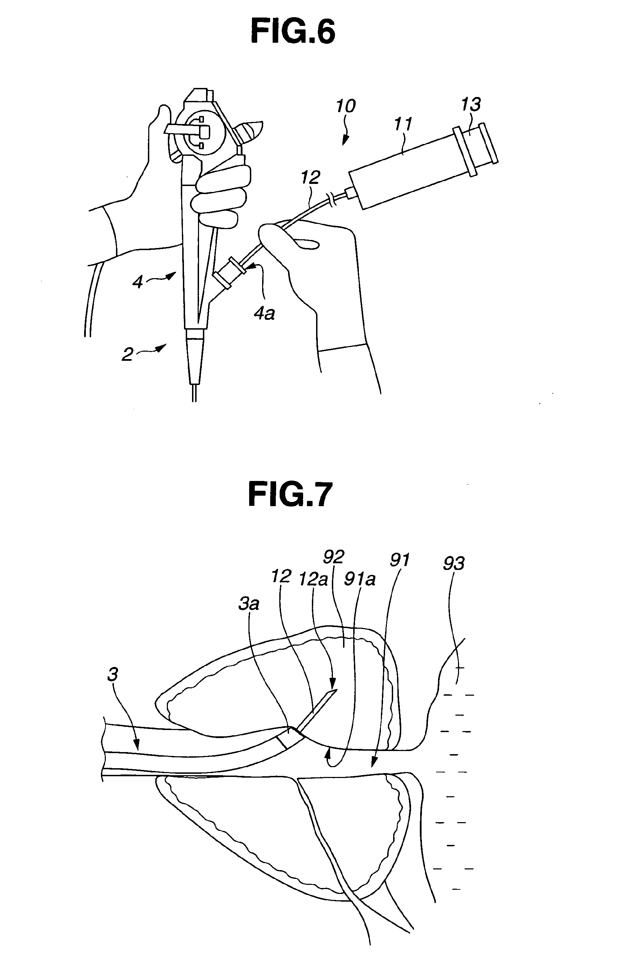

After that, the operator takes the syringe 10 out of the sterile bag 1. Then, as shown in FIG. 6, the operator inserts the needle tube 12 of the syringe 10 into a treatment instrument channel via a treatment instrument insertion port 4a provided on the operation portion 4 of the endoscope 2. Then, after confirming that the needle tube 12 has been inserted to a predetermined position, the operator punctures a needlepoint 12a of the needle tube 12 into the prostate 92, as shown in FIG. 7.

After that, the operator performs pushing-in operation of the inner cylinder 13. Then, the medicinal agent 17 filled in the inner space 16 of the syringe 10 is injected into the prostate 92. As a result, the injection of the medicinal agent 17 into a first position is terminated. At this time, the operator moves the needle tube 12 toward the hand side to dispose the needle tube 12 at a predetermined position in the treatment instrument channel.

Next, the operator moves the insertion portion 3 toward the next indicator determined in advance by appropriately performing insertion operation, twisting operation, and the like of the insertion portion 3. When confirming that the distal end portion 3a has reached the next target region from the endoscope image displayed on the screen of the monitor, the operator punctures the needlepoint 12a of the needle tube 12 to inject the medicinal agent 17 into the prostate 92 as described above. After that, the operator repeatedly performs the above-described hand operation to inject the medicinal agent 17 into the prostate 92. When the medicinal agent 17 is used up, the operator takes a syringe 10 from a new sterile bag 1 to replace the syringe. When the injection of the medicinal agent 17 into the positions determined in advance is completed, the operator extracts the insertion portion 3 of the endoscope 2 from the urethra 91 to terminate the treatment.

Thus, in the syringe for injecting the medicinal agent in which a living bacterium is mixed into a body, the gamma-ray shielding portion is provided on the outer cylinder, the needle portion, or the inner cylinder each of which configures the inner space of the syringe filled with the medicinal agent. This can prevent the gamma rays from reaching the medicinal agent filled in the inner space of the syringe, thereby preventing the bacterium mixed in the medicinal agent from being killed.

Furthermore, the operator can instantly use the syringe for the treatment after taking out the syringe stored in the sterile bag, without performing the work to fill the medicinal agent in the inner space of the syringe.

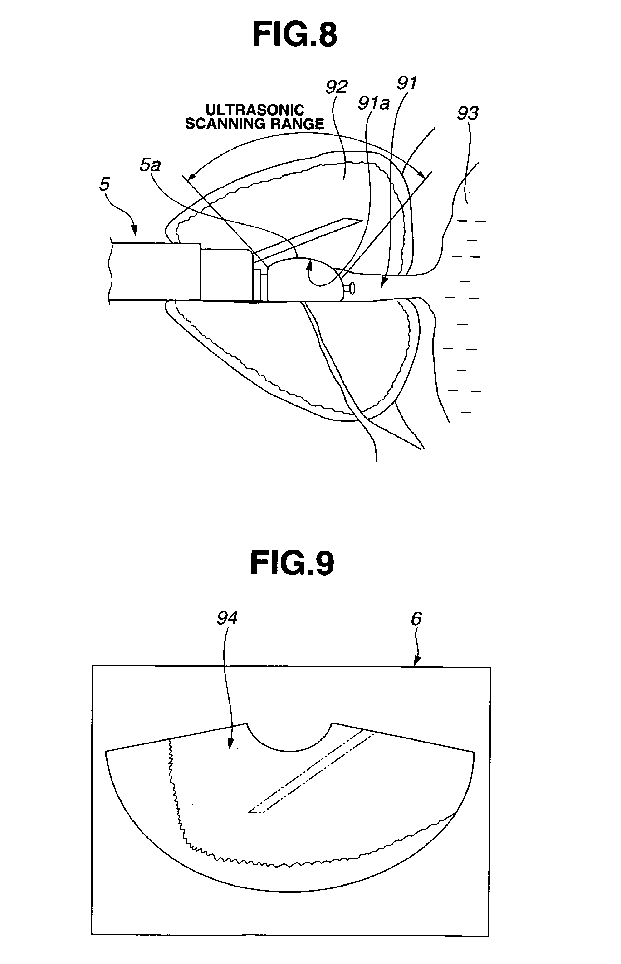

Note that, in the above-described treatment, the medicinal agent 17 is injected by inserting the needle tube 12 of the syringe 10 through the treatment instrument channel provided in the endoscope 2 which includes the observation optical system on the distal end surface of the distal end portion 3a. However, as shown in FIG. 8, the medicinal agent may be injected into the prostate 92 by inserting the needle tube 12 of the syringe 10 through the treatment instrument channel provided in an ultrasound endoscope 5 including a ultrasound transducer portion 5a. This allows the needle tube 12 to be displayed on an ultrasonographic image 94 displayed on the screen 6 of the monitor, as shown in FIG. 9. Therefore, the medicinal agent can be injected after surely grasping the relationship between the region to be treated and the injection position. Accordingly, more effective treatment is possible.

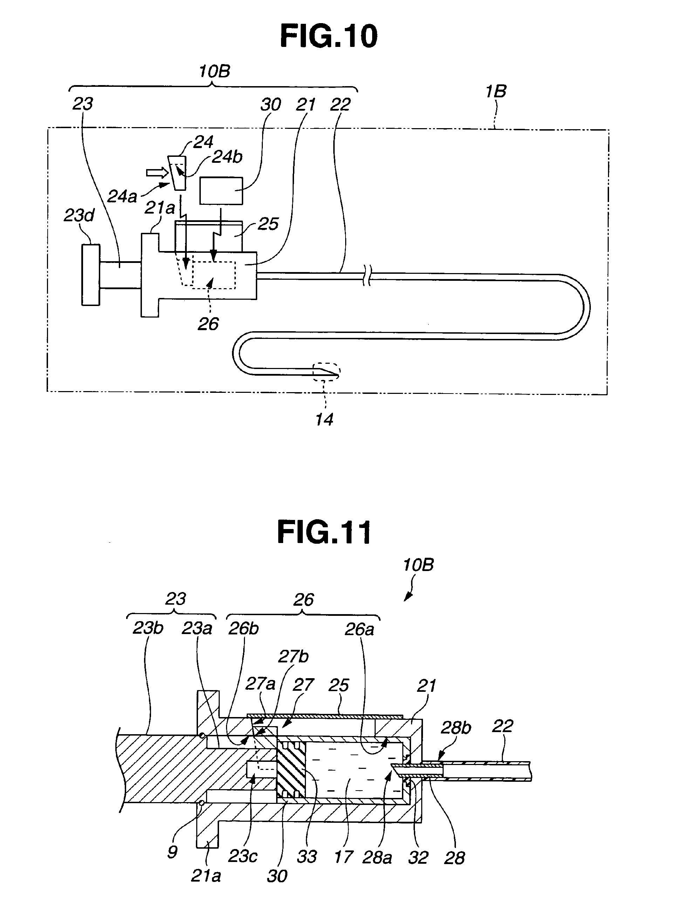

With reference to FIGS. 10 to 12, another configuration of the syringe is described.

The above-described syringes 10, 10A have a configuration in which the medicinal agent 17 is previously filled in the inner space 16 formed in the syringes 10, 10A. In the present embodiment, a syringe 10B is configured by housing inside the outer cylinder 21 a container 30 filled with the medicinal agent 17, as shown in FIG. 10.

As shown in FIGS. 10, 11, the syringe 10B includes the outer cylinder 21, a needle tube 22, an inner cylinder 23, and the container 30. The outer cylinder 21 includes a housing portion 26 serving as a container storing portion in which the container 30 filled with the medicinal agent 17 is housed. To the outer cylinder 21 is integrally provided the needle tube 22 having a bendable flow passage and is slidably disposed the inner cylinder 23 having a pressing portion 23a, for example. In other words, the container 30 filled with the medicinal agent 17 is individually configured.

Note that the reference numerals 21a, 23d, 24, and 25 represent the projection portion for holding, the projection portion for pushing-in operation, a container positioning member, and a lid body, respectively. The container positioning member 24 is in a generally rectangular parallelepiped shape, and has a predetermined surface configured as an inclined surface 24a with a predetermined angle. At a predetermined position of the container positioning member 24 is provided a recessed portion 24b to which the pressing portion 23a of the inner cylinder 23 is loosely disposed. Accordingly, the shape of the container positioning member 24 in an outline arrow direction is in a generally recessed shape. The lid body 25 is rotatably disposed at a predetermined position of the outer cylinder 21 to cover the opening portion.

The outer cylinder 21, the needle tube 22, and the inner cylinder 23 that configure the syringe 10B are configured of transparent resin members, for example. To the outer cylinder 21 are provided the housing portion 26 serving both as a container disposing hole 26a and an inner cylinder slide hole 26b, and a container attaching/detaching opening portion 27 in communication with the housing portion 26 from one side-circumferential surface side.

The housing portion 26 is so formed as to be positioned generally at the center of the outer cylinder 21. At a center position of a bottom surface of the housing portion 26 is integrally provided a rigid needle member 28 made of metal. One end side of the needle member 28 is configured as a sharp needlepoint 28a and is projected into the housing portion 26 by a predetermined amount. The other end side of the needle member 28 is configured as a coupling portion 28b to which the needle tube 22 is mounted, and is projected from the distal end surface of the outer cylinder 21 by a predetermined amount. The needle tube 22 is integrally fixed to the coupling portion by adhesive bonding, for example.

A length dimension and a width dimension of the container attaching/detaching opening portion 27 are set to predetermined dimensions. Specifically, the length dimension is set longer than that of the container 30 by a predetermined dimension, and the width dimension is set wider than that of the outer diameter of the container 30 by a predetermined dimension. This allows the container 30 to be smoothly disposed in the housing portion 26 through the container attaching/detaching opening portion 27. In addition, the proximal end side of the container attaching/detaching opening portion 27 is formed as an inclined surface 27a corresponding to the inclined surface 24a. On the inclined surface 27a is disposed in a closely contacting manner the inclined surface 24a formed on the container positioning member 24.

The inner cylinder 23 is the pressing operation portion and includes the pressing portion 23a and a slide portion 23b. The distal end surface of the pressing portion 23a contacts an exposed surface of a valve 33 configuring the container 30 to press the valve 33, thereby moving the valve 33 toward a bottom surface side. To this end, a diameter dimension of the pressing portion 23a is set to be smaller than an inner diameter dimension of inside of the container 30 by a predetermined dimension. An escape hole 23c is provided at the center of the distal end portion of the pressing portion 23a. In the escape hole 23c, the needle member 28 projecting into the housing portion 26 by a predetermined amount can be disposed.

Meanwhile, the slide portion 23b is disposed in the inner cylinder slide hole 26b of the housing portion 26. An O-ring 9 is disposed without the position thereof being deviated at a predetermined position of the outer circumferential surface of the slide portion 23b. The O-ring 9 adheres tightly to the inner circumferential surface of the inner cylinder slide hole 26b by a predetermined pressing force and contacts a stepped portion 27b formed by the inner cylinder slide hole 26b and the container attaching/detaching opening portion 27.

Accordingly, the slide portion 23b of the inner cylinder 23 is inserted through the inner cylinder slide hole 26b provided to the outer cylinder 21 to dispose the pressing portion 23a in the container disposing hole 26a of the housing portion 26. In this state, when the distal end surface of the pressing portion 23a of the inner cylinder 23 is disposed so as to be close to or contact the bottom surface of the container disposing hole 26a, the needle member 28 is housed in the escape hole 23c. That is, in a state where the inner cylinder 23 is disposed in the outer cylinder 21, the needle member 28 is prevented from being broken or bent by the inner cylinder 23. In addition, in the above-described state, the O-ring 9 is disposed in the container attaching/detaching opening portion 27. Therefore, when the inner cylinder 23 is tried to be extracted from the outer cylinder 21, the O-ring 9 contacts the stepped portion 27b. This prevents the inner cylinder 23 from falling off from the outer cylinder 21.

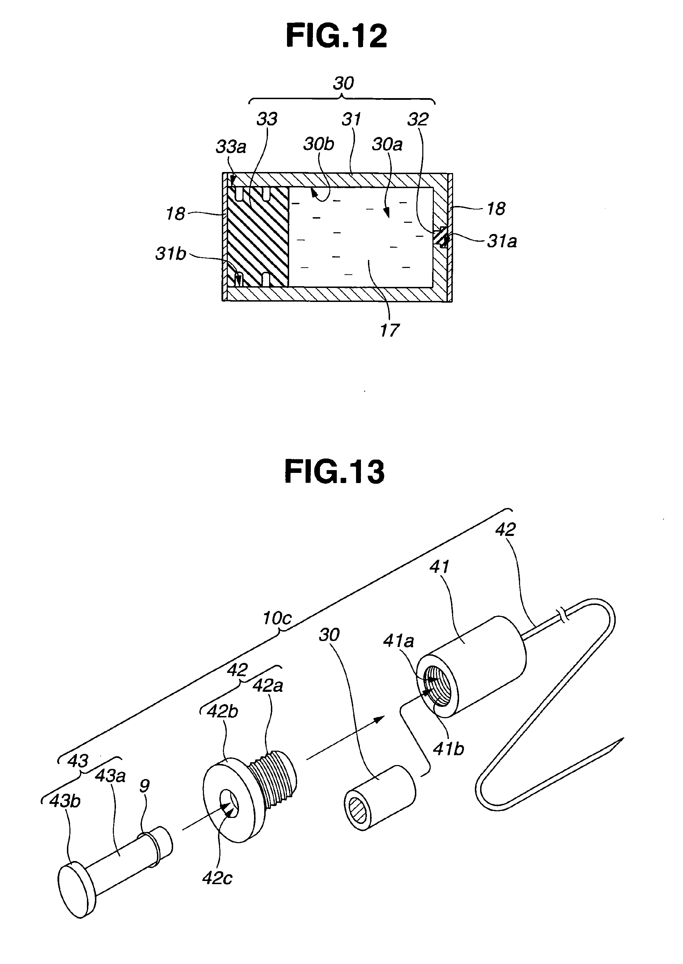

As shown in FIG. 12, the container 30 is a container portion in a cylindrical shape, for example. The container 30 includes a container main body 31, a plug body 32, and the valve 33. The container main body 31 is formed of a metal member for shielding the gamma rays. At a center of a distal end portion of the container main body 31 is provided a central through hole 31 a in a shape of countersink hole to communicate outside and a container space 30a. The central through hole 31 a includes the plug body 32 also serving as a needle tube insertion portion.

Meanwhile, on an opening 31 b side positioned on a proximal end side of the container main body 31 is provided the valve 33 which closes the opening 31 b and moves in a sliding manner with respect to a container inner circumferential surface 30b. The plug body 32 and the valve 33 are made of rubber or resin having a predetermined elastic force. The plug body 32 is firmly and integrally fixed to the central through hole 31 a by adhesive bonding, for example. On the other hand, the valve 33 is fixed integrally to the container inner circumferential surface 30b of the opening 31b at a predetermined adhesive strength by adhesive bonding, for example. Specifically, an adhesive is applied only to a proximal end outer circumferential surface 33a of the valve 33 to adhere the proximal end outer circumferential surface 33a to the container inner circumferential surface 30b. On both end surfaces of the container main body 31 is provided the coating layer 18 made of the gamma-ray shielding member so as to cover the outer surfaces of the plug body 32 and the valve 33.

Then, the syringe 10B is provided in a state where the outer cylinder 21 having the needle tube 22 coupled thereto and the inner cylinder 23 disposed therein, the container positioning member 24, and the container 30 are stored in a sterile bag 1 B shown by the dashed-two dotted line in FIG. 10.

Here, a process for configuring the syringe 10B is described.

First, the outer cylinder 21 is taken out of the sterile bag 1B and the lid body 25 in a closed state is brought to an open state, and thereafter the inner cylinder 23 disposed in the outer cylinder 21 is moved to the proximal end side to dispose the distal end surface of the pressing portion 23a in the inner cylinder slide hole 26b. This allows the housing portion 26 for housing the container 30 to be formed in the outer cylinder 21.

Next, the container 30 is taken out of the sterile bag 1B to be disposed in the housing portion 26 shown by the broken line of FIG. 10. At this time, the plug body 32 configuring the container 30 is brought to face the needle member 28 side. Then, the outer cylinder 21 is inclined so that the side of the projection portion for holding 21a becomes horizontally higher. Then, the container 30 is moved in the housing portion 26 toward the needle member 28 side, thereby allowing the end portion of the container 30 to be disposed in the container disposing hole 26a and also allowing the plug body 32 to be in a contact state with the needlepoint 28a of the needle member 28.

Next, while retaining the state where the plug body 32 contacts the needlepoint 28a of the needle member 28, a narrow side of the container positioning member 24 taken out of the sterile bag 1 B is disposed between the proximal end surface of the container 30 and the inclined surface 27a of the container attaching/detaching opening portion 27. At this time, the inclined surface 24a of the container positioning member 24 is brought in closely contact with the inclined surface 27a. Then, the container positioning member 24 is pushed in toward inside of the housing portion 26. As the container positioning member 24 is pushed into the inside of the housing portion 26, the container 30 is moved toward the needle member 28 side. When the container positioning member 24 has been pushed into the inside of the housing portion 26 in a predetermined state, the container 30 is moved by the inclining angle. This allows the needlepoint 28a of the needle member 28 to pierce the plug body 32, and the needle member 28 is disposed to communicate with the container 30. After that, the lid body 25 is returned to the closed state, thereby configuring the syringe 10B.

In the syringe 10B, the operator performs pushing-in operation to push the inner cylinder 23 toward the needle member 28 side against urging force of the O-ring 9, thereby allowing the distal end surface of the pressing portion 23a to contact the exposed surface of the valve 33. In this contact state, if the operator would like to inject the medicinal agent 17, the operator performs pushing-in operation to further push the inner cylinder 23 ahead. Then, the coating layer 18 is broken, and also adhesive bonding part between the proximal end outer circumferential surface 33a and the container inner circumferential surface 30b is broken. Accordingly, the valve 33 is moved to the needle member 28 side with the movement of the inner cylinder 23, and the medicinal agent 17 filled in the container space 30a is exhausted to the outside from the distal end of the needle tube 22.

The outer cylinder having the needle tube coupled thereto and the inner cylinder disposed therein, the container positioning member, and the container are thus configured, and the container is housed in the housing portion of the outer cylinder, thereby configuring the syringe. Therefore, the gamma-ray shielding portion is provided only to the container in which the medicinal agent is filled. Accordingly, the gamma rays can be prevented from reaching the medicinal agent in the syringe and killing the bacterium mixed in the medicinal agent at the time sterilization, and the syringe can be configured at reduced cost.

In addition, when the medicinal agent in the container is used up during treatment, injection of the medicinal agent can be continued by replacing the container, without the need for replacing the syringe, in other words, without the need for extracting and inserting the needle tube. Moreover, forming the outer cylinder, the needle tube, and the inner cylinder configuring the syringe with a transparent resin member can facilitate confirmation of whether or not the container is stored in the outer cylinder, confirmation of the position of, for example, the distal end surface of the inner cylinder, and confirmation of a flowing state of the medicinal agent. This improves the workability.

Note that a recessed portion in which the O-ring 9 provided to the slide portion 23b is disposed may be provided at a predetermined position on the proximal end side of the inner cylinder slide hole 26b. With this configuration, when the inner cylinder 23 disposed in the outer cylinder 21 is moved to the proximal end side to form in the outer cylinder 21 the housing portion 26 in which the container 30 is housed, the inner cylinder 23 is precisely moved to the predetermined position, thereby enabling the distal end surface of the pressing portion 23a to be disposed in the inner cylinder slide hole 26b.

Furthermore, though the needle member 28 is provided to the outer cylinder 21 and the needle tube 22 is coupled to the needle member 28 in the above-described embodiment, the needle tube 22 may be directly provided at a predetermined position of the outer cylinder 21.

Another configuration of the syringe is described with reference to FIGS. 13 to 16.

Also in the present embodiment, a syringe 10C is configured by housing the container 30 in an outer cylinder 41, similarly as the syringe 10B.

As shown in FIG 13, the syringe 10C includes the outer cylinder 41, the needle tube 22, a container positioning member 42, an inner cylinder 43, and the container 30. In the present embodiment, to the outer cylinder 41 is integrally provided the bendable needle tube 22 and is slidably disposed the inner cylinder 43 including a pressing portion 43a through the container positioning member 42, for example. The outer cylinder 41, the needle tube 22, the container positioning member 42, and the inner cylinder 43, all of which configure the syringe 10C, are made of transparent resin member, for example.

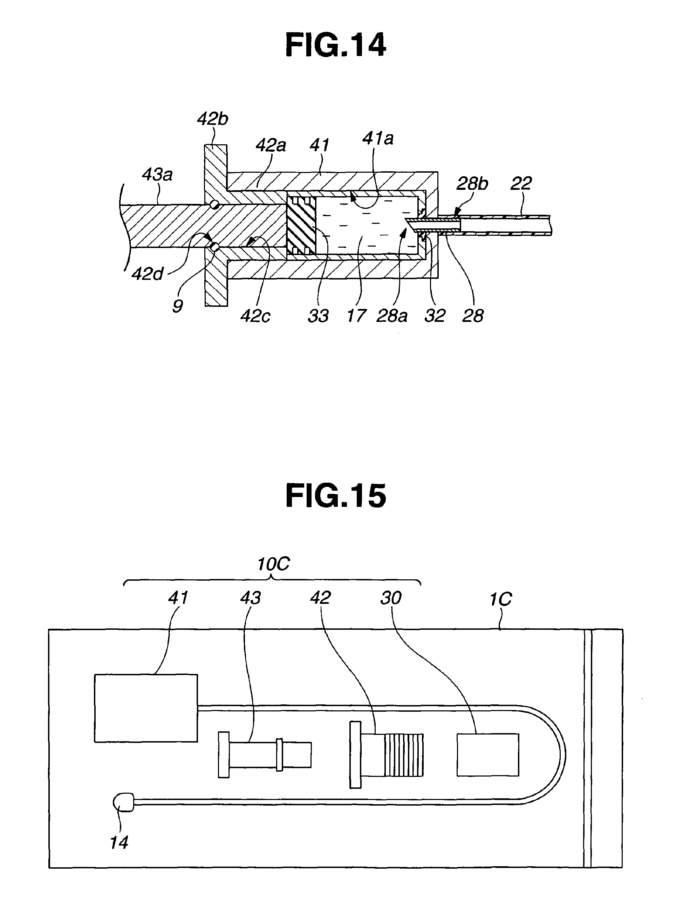

As shown in FIGS. 13, 14, to the outer cylinder 41 is provided a housing hole 41 a serving as the container storing portion having a predetermined depth dimension in which the container 30 filled with the medicinal agent 17 is housed. The housing hole 41 a is provided on an opening-side inner circumferential surface thereof with a female screw portion 41b. Into the female screw portion 41b is screwed a male screw portion (see reference numeral 42a) provided to the container positioning member 42. The housing hole 41 a is provided at a center position on a bottom surface thereof with the needle member 28. One end side of the needle member 28 is configured as a sharp needlepoint 28a and projects into the housing hole 41 a by a predetermined amount. The other end side of the needle member 28 is configured as a coupling portion 28b to which the needle tube 22 is mounted and projects from the distal end surface of the outer cylinder 41 by a predetermined amount. The needle tube 22 is integrally fixed to the coupling portion by adhesive bonding, for example.

The container positioning member 42 is a stepped pipe-shaped member and has the male screw portion 42a on an outer circumference of a small-diameter portion. The male screw portion 42a is configured to screw into the female screw portion 41b provided on the opening side of the housing hole 41a. The container positioning member 42 is provided at one end portion with a projection portion for holding 42b as a large-diameter portion. An outside dimension of the projection portion for holding 42b is set to be larger than that of the outer cylinder 41 taking holding property into account. In a through hole 42c of the container positioning member 42 is disposed by insertion the pressing portion 43a of the inner cylinder 43.

The inner cylinder 43 includes the rod-like pressing portion 43a and a projection portion for pushing-in operation 43b. The pressing portion 43a is so set as to be disposed by insertion in the through hole 42c of the container positioning member 42. A distal end surface of the pressing portion 43a is a pressing surface and an outer circumferential surface of the pressing portion 43a is a sliding surface. The pressing portion 43a is provided at a predetermined position thereof with the O-ring 9. The O-ring 9 is disposed in a circumferential recessed portion 42d provided in the through hole 42c of the container positioning member 42. This prevents the inner cylinder 23 from falling off from the container positioning member 42.

Then, as shown in FIG 15, the syringe 10C is provided in a state where the outer cylinder 41 having the needle tube 22 coupled thereto, the container positioning member 42, the inner cylinder 43, and the container 30 are stored in a sterile bag 1C.

Here, a process for configuring the syringe 10C is simply described.

First, the outer cylinder 41 is taken out of the sterile bag 1C, and also the container 30 is taken out to dispose the container 30 in the housing hole 41a from an opening provided to the outer cylinder 41. At this time, the plug body 32 configuring the container 30 is disposed to face the needle member 28 side. This brings the plug body 32 configuring the container 30 to be in a contact state with the needlepoint 28a of the needle member 28 in the housing hole 41 a.

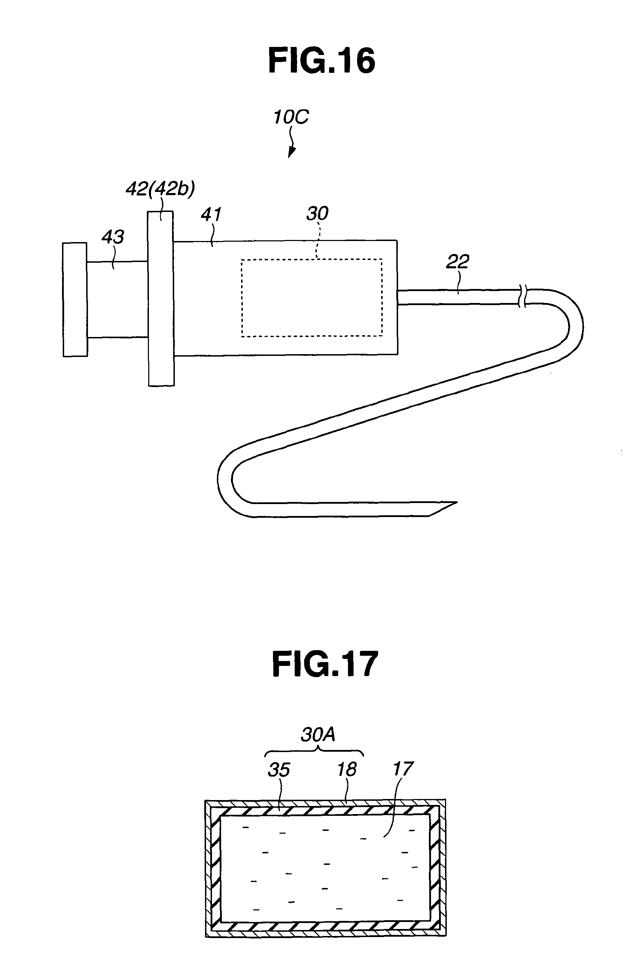

Next, in the state where the plug body 32 contacts the needlepoint 28a of the needle member 28, the male screw portion 42b of the container positioning member 42 taken out of the sterile bag 1C is screwed into the female screw portion 41 b of the housing hole 41 a. Then the container positioning member 42 is rotated in a predetermined direction and the distal end surface of the container positioning member 42 is moved toward a proximal end surface of the container 30. As a result, the distal end surface of the container positioning member 42 contacts the proximal end surface of the container 30. Here, the container positioning member 42 is further rotated. This causes the container 30 to move toward the needle member 28 side with the movement of the container positioning member 42. Then, one surface of the projection portion for holding 21a of the container positioning member 42 contacts the proximal end surface of the outer cylinder 21, thereby allowing the needlepoint 28a of the needle member 28 to pierce the plug body 32, and the needle member 28 is disposed to communicate with the container 30. After that, the pressing portion 43a of the inner cylinder 43 is disposed by insertion in the through hole 42c provided to the container positioning member 42. At this time, the O-ring 9 is disposed in the circumferential recessed portion 42d. Accordingly, the syringe 10C in which the container 30 is stored in the outer cylinder 41 is configured, as shown in FIG 16.

In the syringe 10C, by the operator slightly pushing the inner cylinder 43 ahead to the needle member 28 side, the pressing surface of the pressing portion 43a press-contacts the exposed surface of the valve 33. In this state, if the operator futher pushes in the inner cylinder 43, the coating layer 18 is broken, and also the adhesion-fixed portion of the proximal end outer circumferential surface 33a and the container inner circumferential surface 30b is broken, thereby causing the valve 33 to move toward the needle member 28 side. As a result, the medicinal agent 17 filled in the container space 30a is exhausted to the outside from the distal end of the needle tube 22.

With the syringe 10C, the same working and effects as those of the syringe 10B can be obtained.

Note that, the configuration in which the container 30 is formed in a cylindrical shape with a metal member for shielding the gamma rays is shown. However, the container may be formed of a rubber member or a resin member having elasticity. Specifically, as shown in FIG. 17, a container 30A is configured of a container main body 35 formed of a rubber member having elasticity, for example, and the coating layer 18 of a gamma-ray shielding member covering the outer surface of the container main body 35. The container 30A is suitable for the use in the syringe 10C.

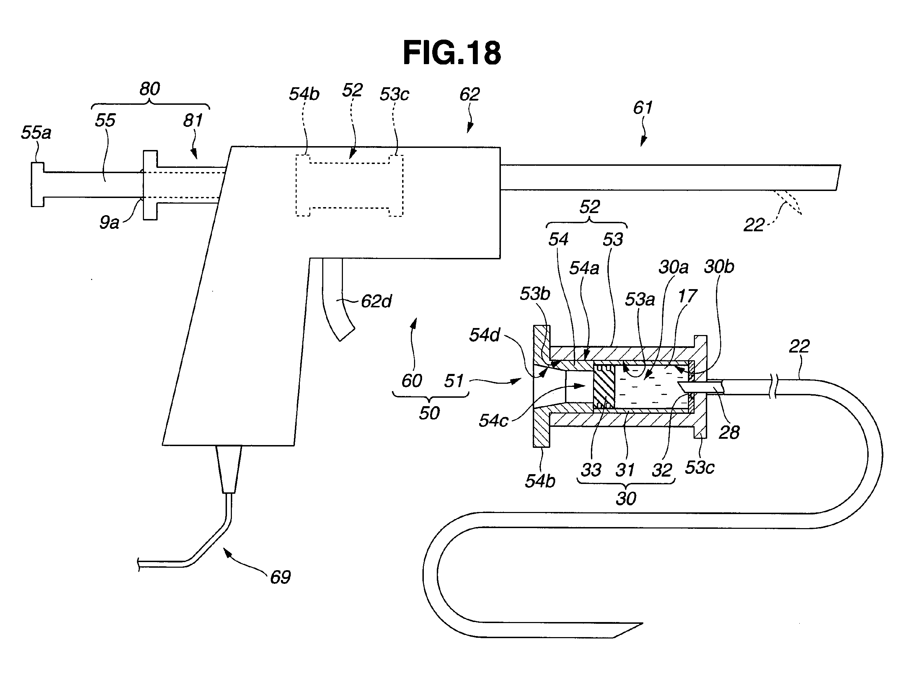

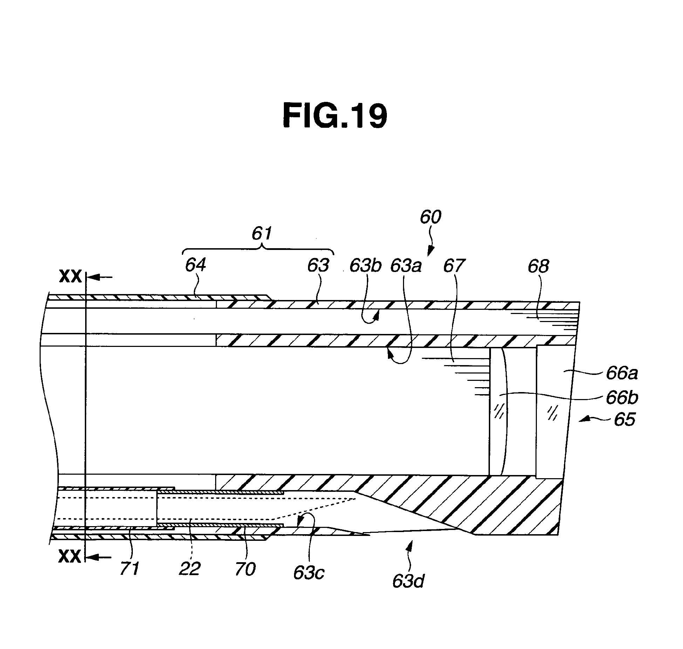

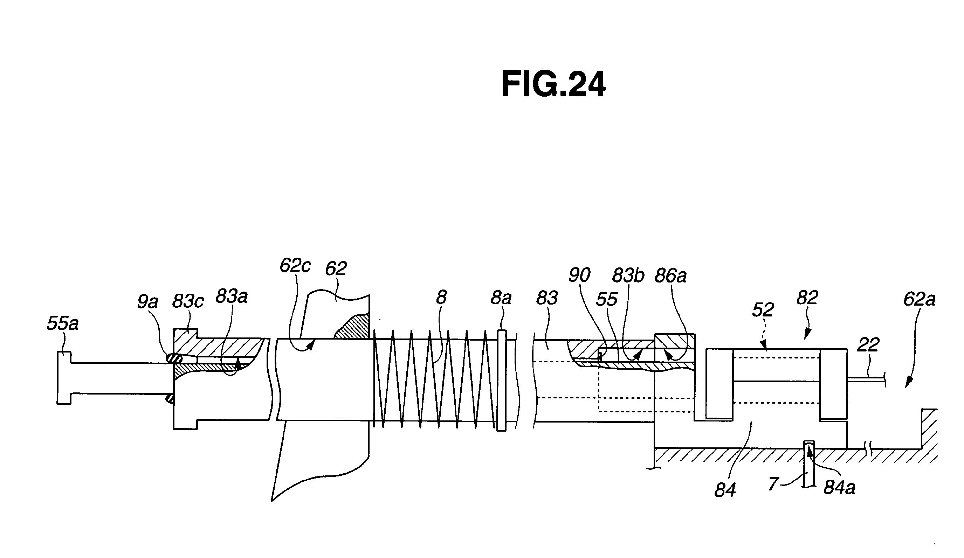

A second embodiment of the present invention is described with reference to FIGS. 18 to 26.

A medical apparatus of the present embodiment shown in FIG. 18 is an injection apparatus 50 for treating prostatic hyperplasia, for example, which is provided with observation means. Specifically, the injection apparatus 50 includes a medicinal agent container 51 from which the needle tube 22 is extended, and an endoscope 60 including the medicinal agent container 51 disposed thereto and an injection operation portion 80 for performing injection operation of medicinal agent. The endoscope 60 includes an observation optical system, to be described later, as observation means.

The medicinal agent container 51 includes the needle tube 22, a case body 52, and the above-described container 30. The case body 52 includes a case main body 53 and a container positioning member 54. The case main body 53 is in a cylindrical shape having a flange portion at one end portion, and has a housing hole 53a serving as the container storing portion having a predetermined depth dimension in which the container 30 is housed. The housing hole 53a is provided on an opening-side inner circumferential surface thereof with a female screw portion 53b. Into the female screw portion 53b is screwed a male screw portion 54a provided to the container positioning member 54. The housing hole 53a includes the needle member 28 provided at a center position on a bottom surface thereof. One end side of the needle member 28 is configured as a sharp needlepoint 28a and projects into the housing hole 53a by a predetermined amount. The other end side of the needle member 28 is configured as a coupling portion to which the needle tube 22 is mounted and projects from the distal end surface of the case main body 53 by a predetermined amount. The needle tube 22 is integrally fixed to the coupling portion by adhesive bonding, for example. The reference numeral 53c represents the flange portion configuring a first positioning projection portion.

The container positioning member 54 is a generally pipe-shaped member having a rod insertion hole 54c and includes a flange portion at one end portion. On outer circumference of the container positioning member 54 is provided the male screw portion 54a. The male screw portion 54a is screwed into the female screw portion 53b provided on the opening side of the inner circumferential surface of the housing hole 53a. The flange portion provided to the container positioning member 54 configures a second positioning projection portion 54b. In the rod insertion hole 54c of the container positioning member 54 is inserted a distal end part of an injection operating rod 55 configuring the injection operation portion 80. On an opening side of the rod insertion hole 54c is provided a tapered opening portion 54d having an opening side formed to have a larger diameter by a predetermined dimension, taking into account an insertability of the injection operating rod 55 into the rod insertion hole 54c. The reference numeral 55a represents a projection portion for pushing-in operation.

Note that, when the case body 52 is configured by fixedly screwing the case main body 53 and the container positioning member 54, positioning projections respectively configured of the first positioning projection portion 53c and the second positioning projection portion 54b have almost the same shape.

As shown in FIGS. 18 to 20, the endoscope 60 mainly includes a rigid insertion portion 61 and an operation portion 62 including a grasping portion, for example. The insertion portion 61 includes a distal end component 63 formed of a rigid resin member, for example, and an insertion portion main body 64 formed likewise of a rigid resin member which are provided in a linked manner. The distal end component 63 is provided with a hole for observation optical system 63a, a hole for illumination optical system 63b, and a treatment instrument channel hole 63c. The hole for observation optical system 63a includes an observation window 66a configuring an observation optical system 65, an optical lens 66b, and an image guide fiber bundle (hereinafter abbreviated as image guide) 67. The hole for illumination optical system 63b includes a light guide fiber bundle (hereinafter abbreviated as light guide) 68 configuring an illumination optical system. Note that illustration of an illumination lens serving also as a cover member disposed on a distal end surface of the light guide fiber bundle is omitted. In the treatment instrument channel hole 63c is disposed by insertion the needle tube 22 extended from the medicinal agent container 51 and the like, for example.

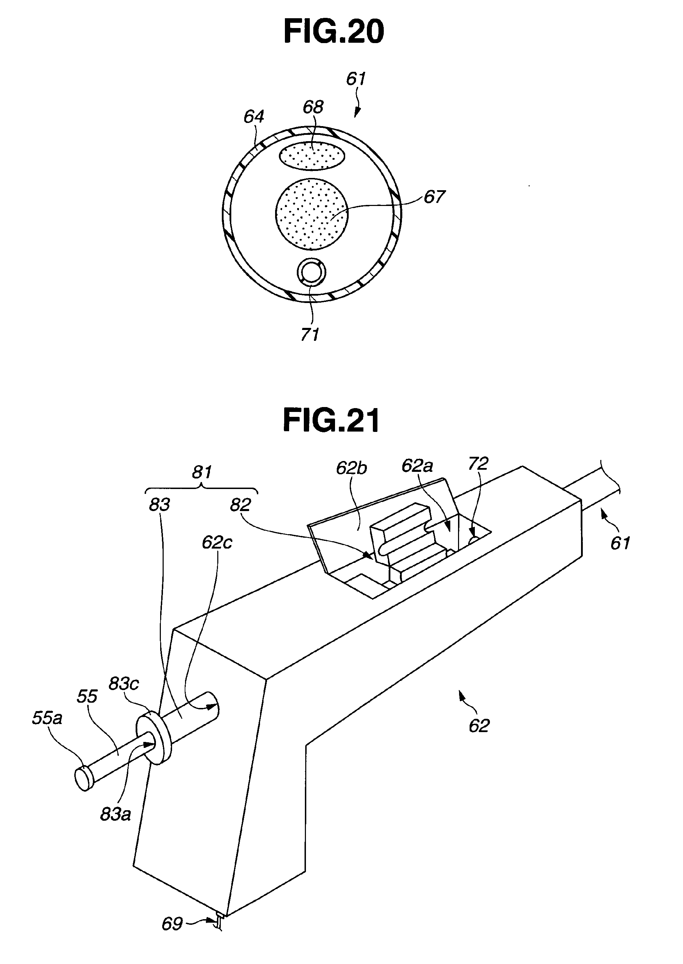

A channel opening 63d of the treatment instrument channel hole 63c is provided on a side surface of the distal end component 63. On a proximal end surface opening of the treatment instrument channel hole 63c is disposed a ferrule member 70. One end portion of the ferrule member 70 projects from a proximal end surface of the distal end component 63, and to the projecting portion is coupled one end portion of a channel member 71 configuring the treatment instrument channel. The other end portion of the channel member 71 is coupled at a predetermined portion in the operation portion 62 so as to be in communication with a treatment instrument insertion port (reference numeral 72 in FIG. 21) provided in the operation portion 62.

The light guide 68 is inserted through inside of the insertion portion main body 64, inside of the operation portion 62, and inside of a universal cord 69 to be connected to a light source apparatus (not shown) as an external apparatus. On the other hand, the image guide 67 is inserted through the inside of the insertion portion main body 64 and disposed so as to face an image pickup surface of an image pickup device not shown provided in the operation portion 62, for example. Therefore, an optical image passed through the observation window 66a and the optical lens 66b to be formed on the image guide 67 and transmitted therethrough is image-formed on the image pickup surface of the image pickup device, and thereafter photoelectrically converted into an electric signal. The electric signal is transmitted to the image pickup apparatus (not shown) as an external apparatus via a signal line extended from the image pickup device to pass through inside of a universal cord 69. The electric signal transmitted to the image pickup apparatus is converted to generate a video signal, and the generated video signal is outputted on the display apparatus (not shown). This causes an endoscope image to be displayed on the screen of the display apparatus.

As shown in FIG 18, and FIGS. 21 to 23, the injection operation portion 80 includes an injection operating rod 55 as the pressing operation portion and a puncture apparatus 81. The puncture apparatus 81 includes a case body disposing table 82, and a tubular disposing table moving rod (hereinafter abbreviated as table moving rod) 83. The case body disposing table 82 is disposed in an apparatus placing hole 62a provided to the operation portion 62. The table moving rod 83 includes a through hole 83a through which the injection operating rod 55 is inserted.

The table moving rod 83 is slidably disposed with respect to an insertion hole 62c provided to the operation portion 62. The table moving rod 83 is guided through the insertion hole 62c to the apparatus placing hole 62a. In the apparatus placing hole 62a is provided a slide holding member (not shown) including a slide groove as a slide portion, for example. The case body disposing table 82 is slidably provided in the slide groove of the slide holding member. In the through hole 83a of the table moving rod 83 is slidably disposed by insertion the injection operating rod 55. The injection operating rod 55 and the table moving rod 83 are set to predetermined dimensions. Then, in a state where the injection operating rod 55 is disposed by insertion in the through hole 83a of the table moving rod 83, when a projection portion for pushing-in operation 55a is brought to contact with a projection portion for holding 83c, a distal end surface of the injection operating rod 55 is projected by a predetermined amount from a distal end surface of the table moving rod 83.

Note that a treatment instrument insertion port 72 is formed at a predetermined position on a distal end side wall surface of the apparatus placing hole 62a. In addition the apparatus placing hole 62a is configured to be closed by a lid member 62b. That is, the lid member 62b is rotatably mounted to the operation portion 62. Furthermore, at a predetermined position in the vicinity of the projection portion for pushing-in operation of the injection operating rod 55 is disposed an O-ring 9a for positioning which has a predetermined urging force, i.e. a predetermined diameter dimension. A reference numeral 62d represents a puncture operating lever (hereinafter abbreviated as lever), and by the lever 62d being operated in a puncture waiting state to be described later, the needle tube 22 is projected from the channel opening 63d.

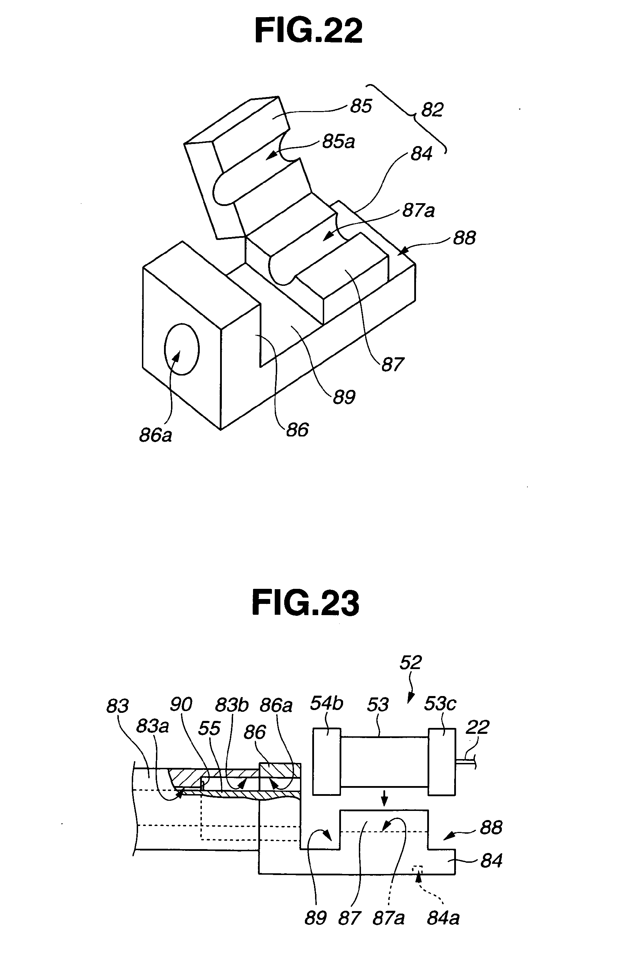

As shown in FIGS. 22, 23, the case body disposing table 82 includes a table main body 84 and a lid body 85. The lid body 85 is rotatably mounted to the table main body 84 by a hinge, for example.

The table main body 84 is provided with a wall portion 86 and a case body placing portion 87. The case body placing portion 87 is provided on the upper surface in the figure with a semicircular placing groove 87a parallel to an axial direction of the insertion portion 61. In the placing groove 87a is placed the case main body 53 configuring the case body 52. A dimension in the axial direction of the case body placing portion 87 is set to be slightly narrower than inner dimension between a first positioning projection portion 53c and a second positioning projection portion 54b which configure the case body 52. Therefore, the case body 52 is stably disposed to the case body placing portion 87 of the table main body 84.

On both side portions of the case body placing portion 87 are respectively provided a stepped portion 88 to which the first positioning projection portion 53c formed on the case body 52 is disposed and a groove portion 89 in which the second positioning projection portion 54b formed on the case body 52 is loosely disposed. Specifically, the groove portion 89 is formed between the wall portion 86 and the case body placing portion 87, and an opening dimension of the groove portion 89 is wider by a predetermined dimension than a width dimension of the second positioning projection portion 54b. Meanwhile, a dimension of the stepped portion 88 is set to approximately the same dimension as a width dimension of the first positioning projection portion 53c. Therefore, in a state where the case main body 53 configuring the case body 52 is placed in the placing groove 87a of the case body placing portion 87, an end surface of the first positioning projection portion 53c and a distal end surface of the table main body 84 are generally coincident with each other. Note that a reference numeral 84a represents a locking hole.

The lid body 85 is in a rectangular parallelepiped shape and includes a semicircular contact groove 85a. The contact groove 85a of the lid body 85 and the placing groove 87a of the case body placing portion 87 are in an opposing positional relationship. Therefore, by the lid body 85 being closed in a state where the case body 52 is placed in the placing groove 87a of the case body placing portion 87, an inner surface of the contact groove 85a is brought to be in a generally contact state with an outer circumferential surface of the case main body 53 which is exposed from the placing groove 87a. This allows the case body 52 to be stably disposed with respect to the case body disposing table 82.

On a proximal end surface side of the wall portion 86 is integrally fixed a distal end portion of the table moving rod 83 by screwing or adhesive bonding. Accordingly, when the table moving rod 83 is advanced and retreated in an axial direction, the case body disposing table 82 is moved following the advancing/retreating movement of the table moving rod 83. On a distal end surface side of the table moving rod 83 is provided a countersink-shaped recessed portion 83b having a predetermined depth dimension. An inner diameter dimension of the recessed portion 83b is larger by a predetermined dimension than a diameter dimension of the through hole 83a. In the recessed portion 83b is disposed an E-ring 90 disposed at a distal end side predetermined position of the injection operating rod 55.

The wall portion 86 is provide with a hole portion 86a having an diameter approximately the same as the inner diameter dimension of the recessed portion 83b provided to the table moving rod 83. A central axis of the hole portion 86a and a central axis of the placing groove 87a forming a semicircle of the case body placing portion 87 are coincident with each other. Therefore, the injection operating rod 55 disposed by insertion in the through hole 83a is to be projected from the distal end surface of the table moving rod 83, with the case body 52 integrally disposed at the case body disposing table 82. Then, a distal end portion of the injection operating rod 55 is inserted into a rod insertion hole 54c through the hole portion 86a on the wall portion 86, and the tapered opening portion 54d of the container positioning member 54 configuring the case body 52. At this time, the E-ring 90 disposed on the operating rod 55 moves inside of the recessed portion 83b and the hole portion 86b. Note that, when the E-ring 90 contacts a bottom surface of the recessed portion 83b, the distal end surface of the injection operating rod 55 is disposed in the hole portion 86a formed on the wall portion 86. In other words, the distal end surface of the injection operating rod 55 is prevented from projecting from the hole portion 86a to the groove portion 89 side.

A process for configuring the injection apparatus 50 is described with reference to FIGS. 18, 21, and 24.

When performing a procedure for treating prostatic hyperplasia, a worker prepares the sterilized endoscope 60 including the injection operation portion 80 as well as the observation optical system 65, and the medicinal agent container 51, from which the needle tube 22 is extended, taken out of the sterile bag.

First, the worker grasps the projection portion for holding 83c of the table moving rod 83 to pull back the table moving rod 83 to a predetermined position against urging force of a spring 8. At this time, because the O-ring 9a and the E-ring 90 are provided at predetermined positions on the outer circumferential surface of the injection operating rod 55, the injection operating rod 55 is moved generally integrally with the table moving rod 83. With the movement of the table moving rod 83, also the case body disposing table 82 disposed in the apparatus placing hole 62a is moved to reach a predetermined position. At this time, a locking nail 7 is disposed in the locking hole 84a provided on a bottom surface of the table main body 84 configuring the case body disposing table 82. This allows the case body disposing table 82 to be retained in a pucture waiting state.

Note that the locking nail 7 is constantly urged to the table main body 84 side by urging means not shown. The puncture waiting state of the case body disposing table 82 is released by operating the lever 62d to move the locking nail 7 to the lower side in the drawing. In other words, the puncture waiting state is continued until the lever 62d is operated. A reference numeral 8a is an E-ring, for example. The E-ring is disposed in a groove (not shown) formed circumferentially at a predetermined position on the outer circumferential surface of the table moving rod 83, thereby disposing the spring 8 in a predetermined urging state.

At this time, the worker confirms whether or not a part of the O-ring 9a is exposed from an end surface of the projection portion for holding 83c. When visual confirmation of the O-ring 9a can not be performed, the worker grasps the projection portion for pushing-in operation 55a to pull the injection operating rod 55 back to the hand side. Then, substantially simultaneously at the time that the E-ring contacts the bottom surface of the recessed portion 83b, a part of the O-ring 9a is brought to be in an exposed state. Thus, the distal end surface of the injection operating rod 55 is disposed at a predetermined position in the hole portion 86a.

Next, the worker causes the lid member 62b provided to the operation portion 62 to be in an open state and also causes the lid body 85 of the case body disposing table 82 provided in the apparatus placing hole 62a to be in an open state. After that, the worker inserts the needle tube 22 extended from the medicinal agent container 51 into the treatment instrument channel hole 63c through the treatment instrument insertion port 72. Then, when the insertion of the needle tube 22 into the treatment instrument channel hole 63c is almost completed, the worker disposes the case body 52 in the placing groove 87a of the case body placing portion 87 provided to the table main body 84 configuring the case body disposing table 82. After that, the worker causes the lid body 85 to be in a closed state and also causes the lid member 62b to be in a closed state. As a result, the injection apparatus 50 in which the case body 52 is provided to the case body disposing table 82 is configured. In the injection apparatus 50, the distal end of the needle tube 22 is disposed in the treatment instrument channel hole 63c without being projected to be exposed from the channel opening 63d.

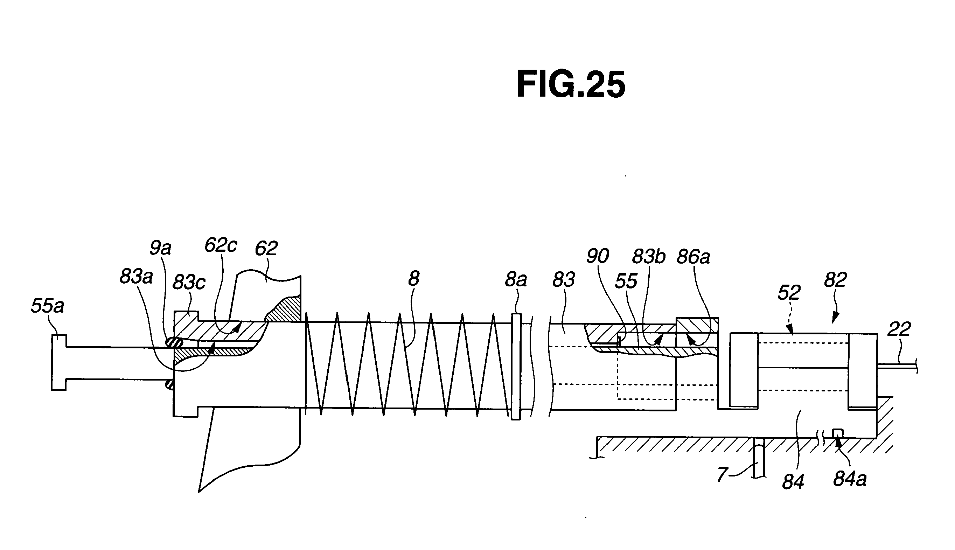

With reference to FIGS. 25 to 27, description is made on transurethral treatment of prostatic hyperplasia using the injection apparatus 50.

At the time of treatment, first an operator inserts the insertion portion 61 of the endoscope 60 in the urethra. Then, the operator confirms whether or not the distal end component 63 reaches the indicator determined in advance, based on an endoscope image displayed on the screen of the monitor. When confirming that the distal end component 63 has reached a target region, the operator performs twisting operation and the like, as needed, to dispose the channel opening 63d at a desired position. Then, the operator operates the lever 62d.

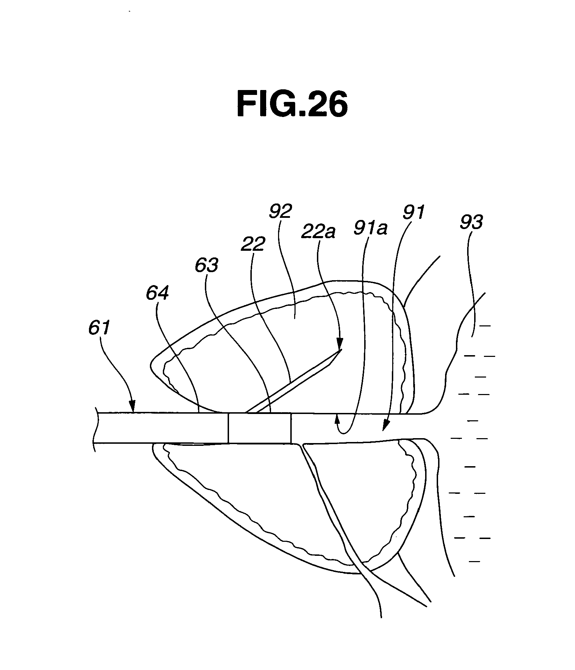

Then, as shown in FIG 25, the locking nail 7 is moved with the operation of the lever 62d, thereby releasing the puncture waiting state. This allows the table moving rod 83 to move integrally with the injection operating rod 55 by the urging force of the spring 8 in the distal end direction. At this time, with the movement of the table moving rod 83, the case body disposing table 82 on which the case body 52 is disposed is also moved. This causes a needlepoint 22a of the needle tube 22 to puncture into the prostate 92, as shown in FIG. 26.

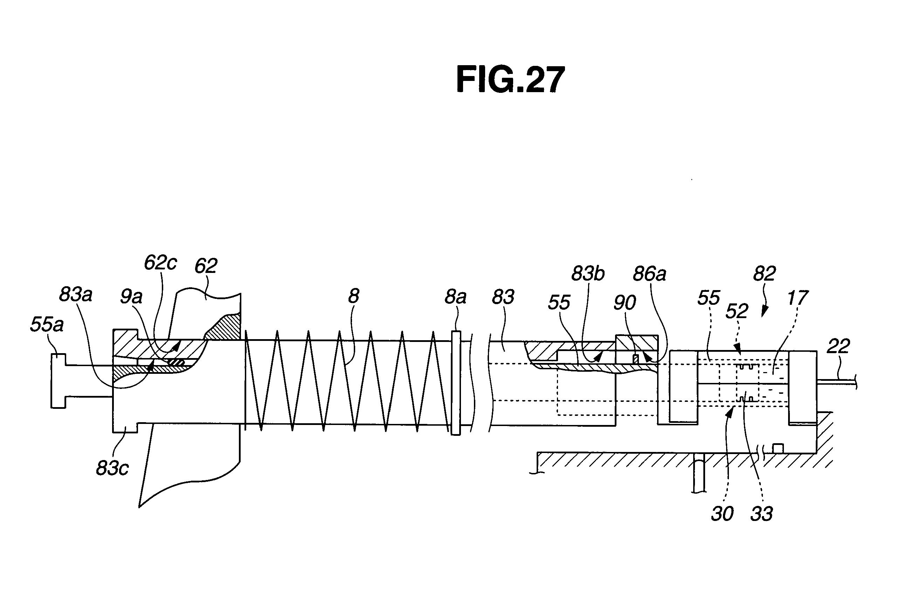

Here, the operator performs pushing-in operation of the injection operating rod 55. Then, the O-ring 9a is pushed into the through hole 83a as shown in FIG 27. This increases an amount of the pushing-in operation force when pushing in the injection operating rod 55. The operator continues pushing-in operation of the injection operating rod 55 against the urging force. This allows the distal end portion of the injection operating rod 55 to be disposed in the rod insertion hole 54c through the tapered opening portion 54d. Then, continuing the pushing-in operation of the injection operating rod 55 brings the distal end surface of the injection operating rod 55 to be in a contact state with an exposed surface of the valve 33. Here, the amount of the pushing-in operation force when pushing in the injection operating rod 55 is further increased. The operator performs the pushing-in operation of the injection operating rod 55 with a larger amount of force. Then, the coating layer 18 is broken, and also the adhesion-fixed portion between the proximal end outer circumferential surface 33a and the container inner circumferential surface 30b is also broken, thereby causing the valve 33 to move toward the needle member 28 side. Then, the medicinal agent 17 filled in the container space 30a of the container 30 is injected into the prostate 92. As a result, the injection of the medicinal agent 17 into a first position is terminated.

After that, the operator grasps the projection portion for holding 83c of the table moving rod 83 and pulls the table moving rod 83 back to a predetermined position against the urging force, to retain the case body disposing table 82 on which the case body 52 is disposed in the puncture waiting state. This allows the needlepoint 22a of the needle tube 22 to be moved in the treatment instrument channel hole 63c.

Next, the operator performs insertion operation, twisting operation and the like of the insertion portion 61, as needed, to move the insertion portion 61 toward the next indicator determined in advance. Then, the operator confirms whether or not the distal end component 63 has reached the next target region based on the endoscope image displayed on the screen of the monitor. When confirming that the distal end component 63 has reached the next target portion, the operator operates the lever 62d, as described above, to puncture the needlepoint 22a of the needle tube 22 and injects the medicinal agent 17 into the prostate 92 by pushing-in operation of the injection operating rod 55. After that, the operator repeatedly performs the above-described hand operation to inject the medicinal agent 17 into the prostate 92. Note that, when the medicinal agent 17 is used up during treatment, the operator replaces the medicinal agent container 51 after use with the medicinal agent container 51 newly taken out of the sterile bag. When the injection of the medicinal agent 17 into points determined in advance is completed, the operator extracts the insertion portion 61 from the urethra 91 to terminate the treatment.

Thus, the injection apparatus for injecting a medicinal agent in which a living bacterium is mixed includes the medicinal agent container with the needle tube, in which the container filled with the medicinal agent is storable in the case body, and the endoscope provided with the observation optical system. The insertion portion of the endoscope includes the treatment instrument channel through which the needle tube of the medicinal agent container is inserted. The operation portion of the endoscope includes the apparatus placing hole in which the case body is disposed. In the apparatus placing hole is slidably provided the case body disposing table. The operation portion is provided with the table moving rod for moving the case body disposing table on which the case body is disposed, and the injection operating rod operated when the medicinal agent filled in the container is injected. Accordingly, after taking out the medicinal agent container stored in the sterile bag, by performing a work for inserting the needle tube through the treatment instrument channel of the insertion portion, and a work for disposing the case body on the case body disposing table provided in the apparatus placing hole, the injection apparatus can be used for treatment. Therefore, after the insertion portion is caused to reach the target region, puncture is instantly performed and the medicinal agent can be injected.

Note that the endoscope 60 is not limited to the above-described configuration, and may employ the configuration shown below.

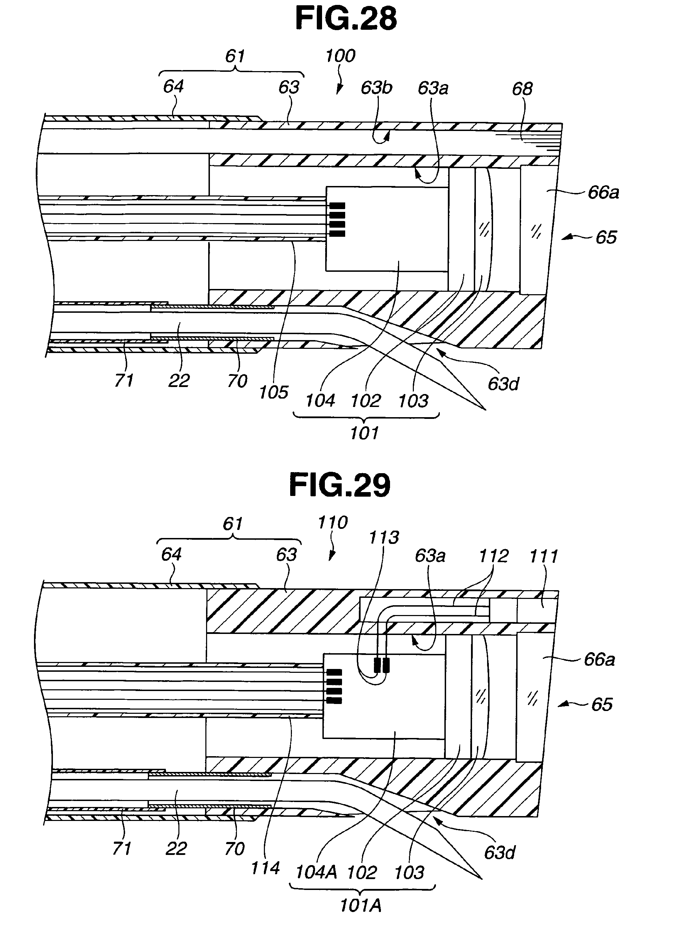

An endoscope 100 shown in FIG. 28 includes an image pickup apparatus 101 to configure an observation optical system 65A, instead of the image guide 67 configuring the observation optical system 65 provided to the endoscope 60. The image pickup apparatus 101 includes an image pickup device 102, an optical lens group 103 disposed on a front face of the image pickup device 102, a substrate 104, and the like. From the image pickup apparatus 101 is extended a signal cable 105 to an image pickup apparatus as an external apparatus. Other configurations are the same as those of the above-described embodiment. The same components are attached with the same reference numerals, and descriptions thereof will be omitted. With such a configuration, the same working and effects as those in the above-described embodiment can be obtained.

An endoscope 110 shown in FIG. 29 is provided with an image pickup apparatus 101A to configure an observation optical system 65A instead of the image guide 67 configuring the observation optical system 65 provided to the endoscope 60, and one or more light emitting elements 111 such as LEDs instead of the light guide 68. An electric cable 112 extended from the light emitting elements 111 is connected to a substrate 104A configuring the image pickup apparatus 101 A. That is, the substrate 104A is additionally provided with a terminal 113 for supplying electric power to the light emitting elements 111. From the image pickup apparatus 101 A is extended a signal line 114 to an image pickup apparatus and a light source apparatus as external apparatuses. Other configurations are the same as those of the endoscope 100. The same components are attached with the same reference numerals, and descriptions thereof will be omitted. With such a configuration, the same working and effects as those in the above-described embodiment can be obtained.

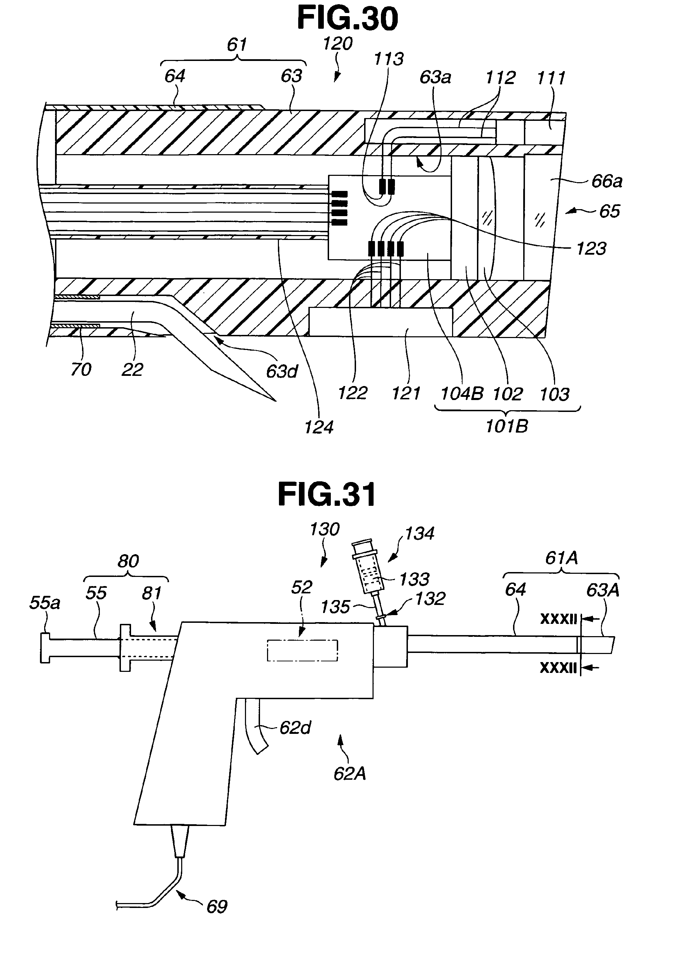

An endoscope 120 shown in FIG. 30 is provided with an ultrasound transducer portion 121 as observation means in addition to the configuration of the endoscope 110 shown in FIG 29. The ultrasound transducer portion 121 is an electronic scanning type in which a plurality of ultrasound elements are aligned, for example. A signal cable 122 is extended from each of the ultrasound elements configuring the ultrasound transducer portion 121. An end portion of each of the signal cables 122 is connected to a substrate 104B configuring an image pickup apparatus 101 B. That is, the substrate 104B is additionally provided with terminals 123 to which the signal cables 122 are connected. From the image pickup apparatus 101 B is extended a signal line 124 to an image pickup apparatus, a light source apparatus, and an ultrasound observation apparatus, which are external apparatuses. Other configurations are the same as those of the endoscope 110. The same components are attached with the same reference numerals, and descriptions thereof will be omitted. With such a configuration, the needle tube is displayed on the ultrasonographic image, thereby enabling the operator to inject the medicinal agent after grasping the region to be treated and the injection positions.

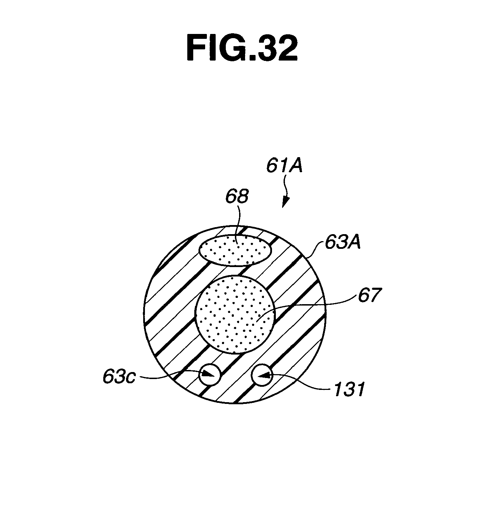

An endoscope 130 shown in FIGS. 31, 32 is provided with a treatment instrument channel hole 131 provided additionally so as to juxtapose to the treatment instrument channel hole 63c, in contrast to the configuration of the endoscope 60 in which one treatment instrument channel hole 63c is provided. The respective channel openings are provided at a predetermined interval on a side surface of the distal end component 63A. A treatment instrument insertion port 132 in communication with the treatment instrument channel hole 131 is provided to a distal end portion of an operation portion 62A, for example. Other configurations are the same as those of the above-described embodiment. The same components are attached with the same reference numerals, and descriptions thereof will be omitted.

With the endoscope 130 thus configured, a needle tube 135 of a syringe 134 filled with a medicinal solution 133 such as analgesic, for example, is inserted in the treatment instrument channel hole 131 through the treatment instrument insertion port 132. This enables the medicinal solution 133 to be injected into the vicinity of a part to be treated of the prostate before injecting the medicinal agent 17 through the needle tube 22 inserted in the treatment instrument channel hole 63c. In other words, smooth injection of the analgesic and the medicinal agent 17 is possible without extracting and inserting the medicinal agent container 51 housing the container 30 filled with the medicinal agent 17 from and to the operation portion 62A.

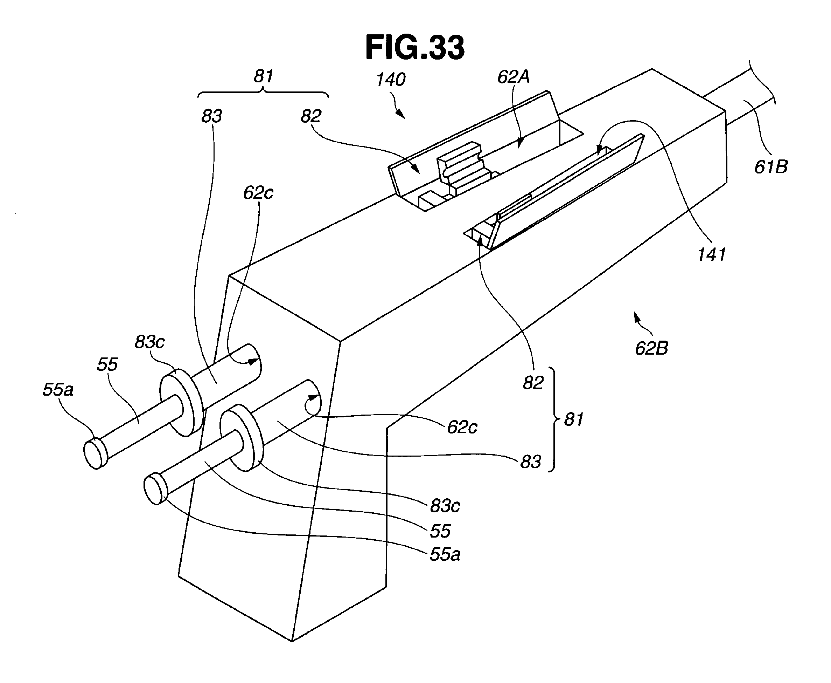

The medicinal solution 133 filled in the syringe 134 is not limited to the analgesic, and may be activator or anti-inflammatory agent. In addition, as shown in FIG. 33, an endoscope 140 may be configured such that a second apparatus placing hole 141, in which the medicinal agent container 51 housing the container 30 filled with the medicinal solution 133 is disposed, is juxtaposed to the operation portion 62B separately from the first apparatus placing hole 62a. In the endoscope 140, the operation portion 62B is further provided with an injection operation portion and a lever.

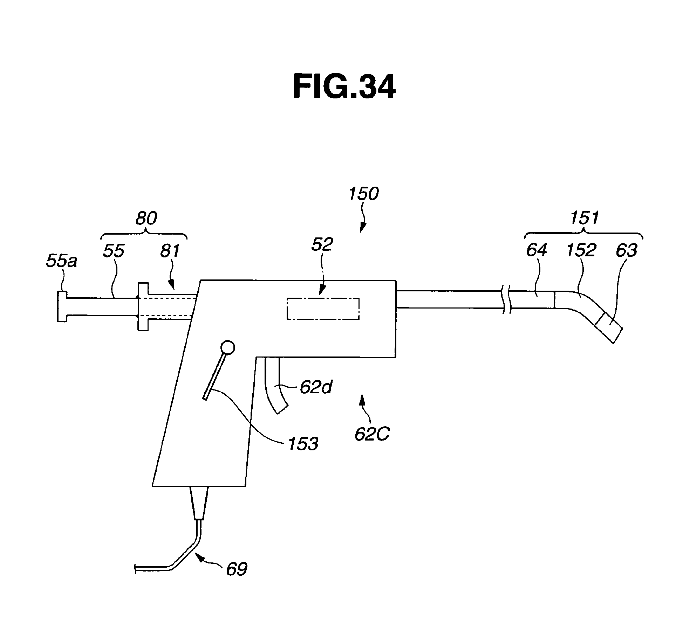

In an endoscope 150 shown in FIG. 34, in contrast to the rigid insertion portion 61 of the endoscope 60, an insertion portion 151 is provided with a bending portion 152 bendable in up/down direction in the drawing, for example. In addition, an operation portion 62C is provided with a bending operation lever 153 for bending the bending portion 152. Other configurations are the same as those of the above-described embodiment. The same components are attached with the same reference numerals, and descriptions thereof will be omitted.

With this configuration, the bending portion 152 is bent by operating the bending operation lever 153, thereby enabling adjustment for smoothly orienting the puncture direction of the needle tube 22 in an optimum direction. Note that the insertion portion 151 may be provided with a flexible tube portion in addition to the bending portion 152.

Note that, the present invention is not limited to the above-described embodiments, but various changes and modifications are possible without departing from the scope of the present invention.

## Claims

1. A medical apparatus comprising:
a container portion including a space portion to be filled with a liquid mixed with a bacterium to be injected into a region to be treated, and a gamma-ray shielding portion for shielding gamma rays irradiated in gamma-ray sterilization;
a needle tube provided with a flow passage through which the liquid passes, the needle tube including one end portion in communication with the space portion of the container portion, and the other end portion to be punctured in the region to be treated; and
a pressing operation portion operated in injecting the liquid filled in the space portion of the container portion into the region to be treated.

2. The medical apparatus according to claim 1, wherein the medical apparatus is a syringe, and the syringe includes:
an outer cylinder serving as the container portion, including a space to be filled with the liquid and a gamma-ray shielding portion for preventing gamma rays from reaching inside of the space;
a needle tube extended from the outer cylinder and to be punctured into a region to be treated; and
an inner cylinder slidably disposed in the outer cylinder to form the space portion between the inner cylinder itself and the outer cylinder, the inner cylinder serving as the pressing operation portion to be pushed in when injecting the liquid filled in the space portion into the region to be treated through the needle tube, the inner cylinder being provided at a part thereof with a gamma-ray shielding portion.

3. The medical apparatus according to claim 2, wherein the needle tube extended from the outer cylinder configuring the syringe is provided with a gamma-ray shielding portion.

4. The medical apparatus according to claim 2 or 3, wherein a liquid-leakage prevention member is detachably provided to a distal end portion of the needle tube, the liquid-leakage prevention member closing a distal end opening of the needle tube to prevent the liquid filled in the space of the outer cylinder from leaking out.

5. The medical apparatus according to any one of claims 1 to 3, wherein the gamma-ray shielding portion is a gamma-ray shielding member forming the outer cylinder, the inner cylinder, and the needle tube, or a coating layer by the gamma-ray shielding member provided on each of a surface of the outer cylinder, a surface of the inner cylinder, and a surface of the needle tube.

6. The medical apparatus according to any one of claims 2 to 4, wherein the syringe is housed in a packaging material for sterilization.

7. The medical apparatus according to claim 1, wherein the medical apparatus is a syringe, and the syringe includes:
a container serving as the container portion, including a container space filled with a liquid containing a bacterium to be injected into a region to be treated and a gamma-ray shielding portion for shielding gamma rays irradiated in gamma-ray sterilization;
an outer cylinder including: a container storing portion in which the container is detachably housed; and a needle member including a flow passage through which a liquid filled in the container passes, the needle member being disposed to communicate with the container space of the container; and
an inner cylinder slidably disposed in the outer cylinder, the inner cylinder being the pressing operation portion to be pushed in when injecting, through the needle tube, the liquid filled in the container space of the container into a region to be treated.

8. The medical apparatus according to claim 7, wherein the syringe further includes a positioning member for disposing the container at a predetermined position of the container storing portion.

9. The medical apparatus according to claim 7 or 8, wherein the syringe is housed in a packaging material for sterilization.

10. The medical apparatus according to claim 1, wherein the medical apparatus is an injection apparatus, and the injection apparatus includes:
a medicinal agent container including: a container storing portion in which a container as the container portion having a container space to be filled with a liquid containing a bacterium is stored; and a needle tube having a flow passage through which the liquid filled in the container housed in the container storing portion passes; and
an endoscope including: observation means at a distal end portion thereof; and at an operation portion thereof provided in a linked manner with an insertion portion having a treatment instrument channel through which the needle tube is inserted, a puncture apparatus, to which the medicinal agent container is detachably disposed, for puncturing a needle tube provided to the medicinal agent container into a region to be treated, and an injection operation portion as the pressing operation portion to be pushed in when injecting, through the needle tube, the liquid filled in the medicinal agent container disposed to the puncture apparatus into a region to be treated.

11. The medical apparatus according to claim 10, wherein the insertion portion includes at least one treatment instrument channel hole through which the needle tube is inserted.

12. The medical apparatus according to claim 10, wherein the endoscope is further provided at the operation portion with a locking nail for retaining the case body disposing table in a puncture waiting state, and a puncture operation lever for releasing the puncture waiting state of the case body disposing table retained in the puncture waiting state.

13. The medical apparatus according to claim 10, wherein the observation means is an observation optical system for obtaining an endoscope image.

14. The medical apparatus according to claim 10, wherein the observation means is an ultrasound transducer portion for obtaining an ultrasonographic image.

15. The medical apparatus according to claim 10, wherein, in a configuration in which two treatment instrument channel holes are provided to the insertion portion, a needle tube for injecting a liquid containing a bacterium is disposed in one of the treatment instrument channel holes, and a needle tube for injecting medicinal agent such as analgesic, activator, or anti-inflammatory agent in the other of the treatment instrument channel holes.

16. The medical apparatus according to claim 10, wherein, in a configuration in which two treatment instrument channel holes are provided to the insertion portion, the operation portion includes: two puncture apparatuses, to which the medicinal agent containers are detachably disposed, respectively, for puncturing the needle tube disposed to the medicinal agent container into a region to be treated; and two injection operation portions as the pressing operation portions to be pushed in when injecting, through the needle tubes, the liquid filled in the medicinal agent containers disposed to the puncture apparatuses into a region to be treated.
